# EUROPEAN PATENT SPECIFICATION

(11) **EP 4 209 249 B1**
(45) Date of publication and mention of the grant of the patent: **16.04.2025**
(21) Application number: 21864165.2
(22) Date of filing: 23.08.2021
(51) Int. Cl.: A61N 1/04, A61N 1/36, A45D 44/00, A45D 44/22, A61N 1/32

(54) **COSMETIC MASK, AND COSMETIC ELECTRIC STIMULATOR**
KOSMETISCHE MASKE UND ELEKTRISCHER KOSMETIKSTIMULATOR
MASQUE COSMÉTIQUE ET STIMULATEUR ÉLECTRIQUE COSMÉTIQUE

(30) Priority: 02.09.2020 JP 2020147850
(43) Date of publication of application: 12.07.2023
(73) Proprietor: Ya-Man Ltd., Tokyo 135-0045 (JP)
(72) Inventor: Kojima Hideaki, Tokyo 135-0045 (JP); Fukui Soichiro, Tokyo 135-0045 (JP)
(74) Representative: Gosdin, Carstensen & Partner Patentanwälte Partnerschaftgesellschaft mbB
(86) International application number: PCT/JP2021/030800
(87) International publication number: WO 2022/050107

(56) References cited:
- WO-A1-2015/040583
- WO-A1-2016/009463
- WO-A1-2019/093023
- WO-A1-2019/093042
- CN-A- 108 159 562
- CN-A- 111 437 089
- JP-A- 2023 007 120
- KR-A- 20150 124 239
- KR-B1- 101 741 067

## Description

### Technical Field

The present invention relates to a cosmetic mask that is configured to be worn on the face of a user to give stimulation and a cosmetic electric stimulator, and particularly relates to a technique that makes it easy to wear the cosmetic mask and improves a cosmetic effect.

### Background Art

A mask for applying electric stimulation to various muscles of a user's face is conventionally known.

A generic cosmetic mask is known from WO 2019/093023 A1.

For example, Patent Literature 1 discloses a cosmetic mask including a mask body section covering a face, the mask body section is made of silicon, have openings at positions of eyes, a nose, and a mouth, and have an electrode section on an inner surface of the mask body.

Further, Patent Literature 2 discloses a technique including elastic base material covering a face, the elastic base material made of silicon, have openings at positions of eyes, a nose, and a mouth, and have a stimulation electrode section at location to which electric stimulation is applied, and states that the technique is effective for face lift-up.

Patent Literature 3 discloses a cosmetic device including an operation member covering a face. It is described that the operation member has a cover section made of silicon, a conductor in which an electrode is formed around the cheek, and openings at positions of eyes, the nose, and the mouth.

Patent Literature 4 discloses a treatment mask including a mask body covering a face, having openings at positions of eyes, a nose, and a mouth, and having an electrode on an inner surface of the mask body. Further, it describes that the mask body is made of a silicone rubber material, and that the mask body deforms under its own weight to uniformly adhere to the skin.

Patent Literature 5 discloses a prior art partly by the applicant of the present application and a configuration of a cosmetic mask that is configured to be worn on the face of a user and cover at least the cheek, the cosmetic mask including a pair of stimulators that provides electric or ultrasonic stimulation to a location corresponding to at least one of the zygomatic muscle and the masseter muscle of the user, and a stimulator fitting section for fitting the stimulators on the positions of the cheeks, in which at least a part of the stimulators is detachably attached. In this literature, it is proposed to include left and right attachments to be attached while applying a tensile force to the left and right sides of the face for wearing the mask, and upper and lower attachments to be attached while applying a tensile force from the lower surface of the mandible bone to the top of the head through both sides of the face.

Other solutions are known from KR 101 741 067 B1, from JP 2023 007120 A and from CN 111 437 089 A.

### Citation List - Patent Literature

Patent Literature 1: WO 2016/009463 A1
Patent Literature 2: JP 2017-108758 A
Patent Literature 3: JP 2013-081606 A
Patent Literature 4: JP 3082187 U
Patent Literature 5: WO 2019/093042 A

### Summary of Invention

### Technical Problem

According to the above-described conventional techniques, it is known that openings are provided at the positions of eyes, a nose, and a mouth, and an electrode that electrically stimulates the cheek area is provided, but it is not conceived to correspond to the positions and characteristics of individual muscles. In particular, the electrodes on the cheek section are too large, and they may give inappropriate stimulation. Furthermore, inadequate elasticity of the mask failed to give appropriate tightening effect when worn, and masks having both wearability and a tightening effect have not been provided.

Although the technique of Patent Literature 5 attempts to solve these problems, fixation by the two attachments is slightly complicated, and face outline lift-up effect and the range of stimulation and strength thereof left a room for improvement.

In view of the above problems of the prior art, an object of the present invention is to provide a cosmetic mask that can be easily worn and removed and has an enhanced cosmetic effect, and an electric stimulator applicable to the cosmetic mask.

### Solution to Problem

In order to solve the above problems, the present invention provides a cosmetic mask according to the attached claims.

### Advantageous Effects of Invention

The cosmetic mask is formed as an abbreviated band that wraps around the occiput from the chin tip through the right and left cheeks, allowing the cosmetic mask to be applied easily in a single operation and precisely aligned with the prescribed location to be stimulated by fitting in the chin tip.

In the meantime, wrap-around section splits at the occiput and made detachable and attachable with a hook-and-loop fastener, allowing for single operation.

Configuring each width of the upper cheek section and the wrap-around section in lateral view to be all substantially equal to the width from the lower edge of the mouth periphery to the neck-side end of the mandible section from the lateral view of the face contributes to comfortable wearability as specific locations become pressure free and improves aesthetics of the appearance.

As described above, the present invention can provide a cosmetic mask that can be easily attached and detached, aesthetic, and has an enhanced cosmetic effect, and an electric stimulator applicable to the cosmetic mask.

### Brief Description of Drawings

Fig. 1 is a front view of a mask body according to the present invention.
Fig. 2 is a lateral view of the mask body according to the present invention.
Fig. 3 is a front view of a stimulator according to the present invention.
Fig. 4 is a rear view of the stimulator according to the present invention.
Fig. 5 is a cross-sectional view of the stimulator according to the present invention.
Fig. 6 is an explanatory view illustrating a use state of a cosmetic mask according to the present invention.
Fig. 7 is an explanatory view illustrating an aspect of a stimulator attachment.
Fig. 8 is an explanatory view illustrating a method for forming the mask body according to the present invention.

### Description of Embodiments

Hereinafter, embodiments of the present invention will be described with reference to examples illustrated in the drawings. Note that the embodiments are not limited to the following.

A cosmetic mask of the present invention is a cosmetic mask which is configured to be worn on the face of a user, and to cover at least the cheeks. For example, the cosmetic mask is preferably made of a flexible elastic material such as silicon or natural rubber. In particular, silicon is optimal for use in contact with the skin and in terms of tightening strength, as it is widely used for medical purposes.

In the following examples, a stimulator including electrodes that electrically stimulates the zygomatic muscle and the masseter muscle is illustrated as the stimulator, but the embodiment is not limited thereto. Although the configuration in which the electrical stimulation is applied as a zygomatic muscle stimulator or a masseter muscle stimulator has been described, ultrasonic stimulation can be applied as a cosmetic method as known, and ultrasonic waves may be used as the stimulator of the present invention. It may be configured to provide electrical stimulation and ultrasonic stimulation therapy.

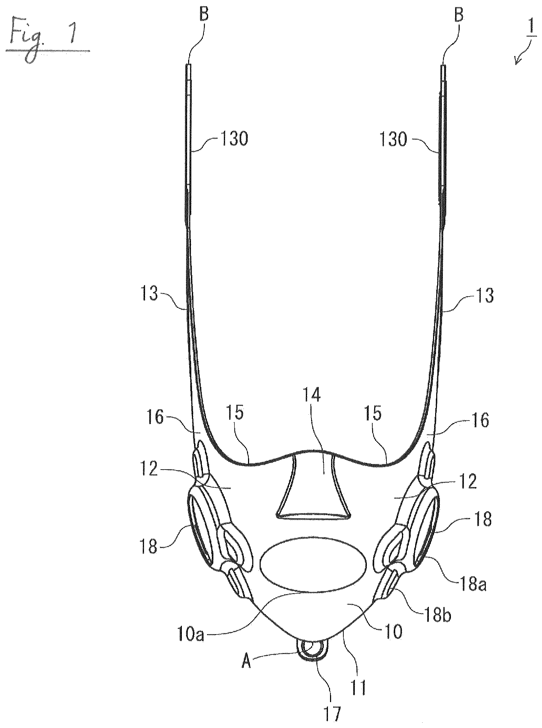

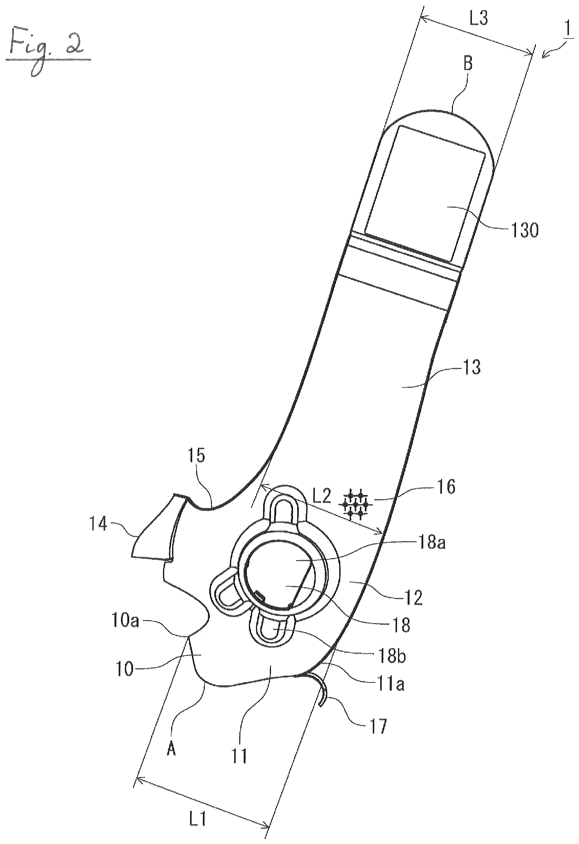

Figs. 1 and 2 are a front view and a lateral view of a mask body (1) according to the present invention, respectively. The mask body (1) is integrally molded with silicon, and is formed in an elastic band shape to be wrapped around the occiput (B) from a chin tip (A) through left and right cheeks. The mask body (1) includes a mouth periphery (10) that covers a location corresponding to the user's mouth with an opening, a mandible section (11) that covers the chin and at least a part of a lower surface of the mandible, a cheek section (12) that covers a part of cheek, and a wrap-around section (13) that is wrapped around the lateral sides and the occiput.

The mouth periphery (10) with a predetermined width is an opening around the red lips and is formed to contact the skin around the periphery. In the present embodiment, the distance from the chin tip (A) to the lower edge of the opening is about 33 mm and the distance to the upper edge is about 67 mm. However, for better wearability of the mask body (1) and applying appropriate pressure on the skin, the distance to the lower edge is preferably in a range of 25 mm to 50 mm, and to the upper edge is preferably in a range of 55 mm to 85 mm.

Further, the nosepiece (14) may be formed in a three-dimensional form that conforms to the shape of the nose. By fitting the nosepiece (14) and the chin tip (A) to the face, the cosmetic mask can be easily centered when worn, the upper and lower parts fit the convex shape of the face at the front side of the face or the better fit of the mask.

When a nosepiece (14) is provided, a concave upper edge (15) is formed from the top edge of the nosepiece (14) to both sides to avoid the eyes, and extends to the wrap-around section (13) by forming a smooth curve.

The continuous shape from the nosepiece (14) to the upper edge (15) and the wrap-around section (13) improves aesthetics giving a neat impression and covers the lower half of the face widely, preventing slack and protrusion of the skin from the mask.

In the present invention, a configuration without a nosepiece (14) is feasible, and a configuration of a mask body (1) can be wrapped around the face from the chin tip(A) to the occiput.

When a part continuing to the cheek section (12) from the nosepiece (14) is removed, each of widths (L2) and (L3) of the cheek section (12) and the wrap-around section (13) in lateral view is substantially equal to a width (L1) from a lower edge (10a) of the opening in the mouth periphery (10) to a neck-side end (11a) of the mandible section in lateral view of the face.

According to the present configuration, the mask is less likely to be twisted or budge because the mask body (1) is pulled up straight from the mandible section (11) to the wrap-around section (13) via the cheek section (12), and its substantially equal width distributes pressure over the skin surface, that prevent skin wrinkle and bring a good cosmetic effect.

In the present embodiment, an ear section (16) corresponding to the ear also ensures ventilation with a plurality of small pores. Conventionally, a large opening is provided in the ear section that surrounds the ear was well-known, but a narrow section might get entangled in hair or press on the carotid artery. The configuration of the present invention has a sufficiently wide band shape that solves these issues.

In the mandible section (11), the distance from the chin tip (A) to the neck-side end (11a) of the mandible section is about 57 mm. In the present invention, mask body (1) is held firmly by the tensile force due to elasticity between the chin and the occiput, the mandible section (11) is in contact with a sufficient width, so that the user feels less pressure. From this viewpoint, the length of the location is preferably 40 mm or more.

In the present invention, the mask body (1) is configured to have an elastic band shape and be substantially straight, but preferably, the wrap-around section (13) may be slightly tilted forward from a center line passing through the mouth periphery (10), the mandible section (11), and the chin tip(A) of the cheek section (12).

In the present embodiment, an angle between a virtual line passing through the center of a stimulator fitting section (18) from the chin tip(A) and a virtual line passing through the center of the wrap-around section (13) in the width direction is about 165 degrees. By setting the angle to 150 degrees to 175 degrees as a suitable range, the position of the mask body (1) is appropriately attached so that it may be pulled up almost straight from the chin tip(A) and prevents the mask body (1) from sliding down from the occiput to the neck.

Further, the angle between the virtual line passing through the center of the wrap-around section (13) in the direction of width and the surface along the lower side of the mandible section (11) is 100 degrees to 130 degrees, and preferably about 118 degrees.

A tab (17) can be provided at the end (11a) of the mandible section (11) on the neck side to pull it toward the neck to wear the cosmetic mask. The tab (17) is integrally molded and made of silicon, and to be folded frontward to form a bow shape as shown in the drawing. The users can hold the inner peripheral side by pinching it between the index and thumb, and pull it downward and toward the neck, and firmly fit the jaw into the chin tip section (A).

The upper end of the wrap-around section (13) is split at the top of the occiput and is fastened by a hook-and-loop fastener (130). Therefore, the user holds both left and right sides of the wrap-around section (13) and turns the wrap-around section (13) to the occiput, and fastens the hook-and-loop fasteners (130) holding it with an appropriate tensile force and wear the present cosmetic mask.

Note that the fastening mean is not limited to a hook-and-loop fastener, and may be a hook, a single-operation buckle, or the like. Further, the entire mask body (1) may be configured to form an annular shape and allow it to be fitted using its elastic stretchability.

The stimulator fitting section (18) for fitting a stimulator (2) is formed in the cheek section (12). The stimulator fitting section (18) includes an opening (18a) in which the stimulator (2) is fit by partially exposing the upper side of the stimulator (2) and an electrode fitting section (18b) formed to fit only on the skin contact surface.

The configuration of the stimulator fitting section (18) is not limited to have an opening as in the present embodiment, but it may be configured to have an engagement section that engages with the skin contact surface of the mask body (1).

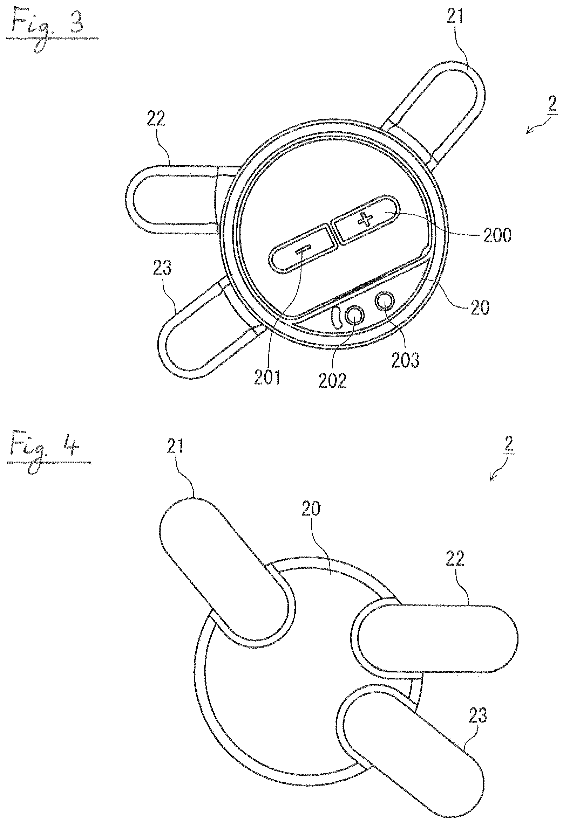

Figs. 3, 4, and 5 are front view, back view, and cross-sectional view of the stimulator (2) according to the present invention. The stimulator (2) includes a current generator (20) provided with a power source and adjustment buttons (200) and (201) on the outer surface when the cosmetic mask is worn, and three electrode sections (21), (22), and (23) extending radially from the current generator.

The current generator (20) is a circle with a diameter of about 50 mm (suitable range: 40 mm to 60 mm), and the electrode sections (21), (22), and (23) are rounded rectangles with a width of about 12 mm (suitable range: 10 mm to 15 mm), a length of about 35 mm, and a length of a section in contact with the skin of about 22 mm (suitable range: 15 mm to 35 mm), respectively. The shape of the current generator (20) is not limited to substantial triangle, and may be any shape such as circle, oval, rectangular, and hexagonal. Further, the shape of the electrode sections may be any shape such as circular, oval, rectangular, and rhombic.

The stimulator (2) is configured to electrically stimulate, and includes, inside the current generator (20), a controller (not illustrated) that controls a current and power supply terminal (202) and (203) that can charge a rechargeable battery (not illustrated) and supply power. Comfortable and low-cost use is attained without using power cables or external battery, while it can be powered by an external power supply or battery.

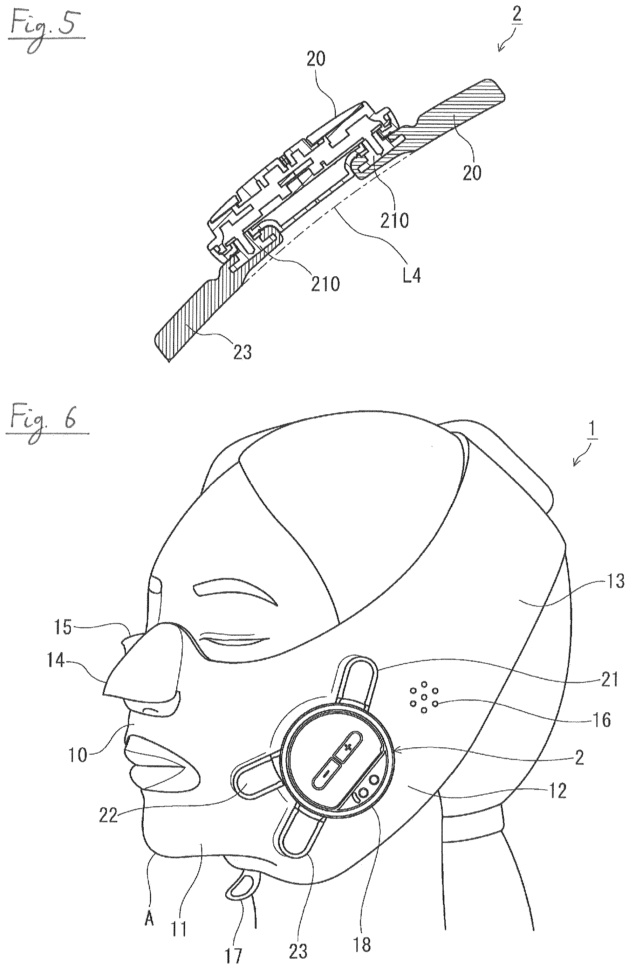

The electrode sections (21), (22), and (23) made of conductive silicon, are electrically connected to an internal electrode (210) in the current generator (20) as shown in Fig. 5, and galvanize the skin from the surface contacted with the skin. The electrode sections (21), (22), and (23) are flexible and fit the skin so that they can be spread when the stimulator (2) is pressed against the skin, and the virtual line (L4) between the electrode sections is similarly curved. Between the electrodes, consideration is also given to avoiding discomfort or heat when the current generator (20) made of a material such as hard plastic directly touches the user's skin because the surface of the current generator (20) that contacts the skin is set back from the virtual line (L4).

The zygomaticus muscles, including the major and minor zygomaticus muscles, which have the function of lifting slack cheeks and nasolabial folds, and in this embodiment, both areas are galvanized to obtain a training effect. Sagging corners of the mouth and loss of muscular strength due to aging, as well as nasolabial fold, double jaw, and sagging face outline, can be improved by training the zygomaticus muscles.

The masseter muscle is a muscle that controls the biting force, is prone to fatigue due to chewing and clenching of teeth. Further, if the masseter muscle is over-developed by bruxism, the user's face outline becomes unbalanced, like square-jawed. In the same way, if the electrical stimulation improperly trains masseter muscles, the face outline may be unbalanced as well, and brings an adverse cosmetic effect.

A low-frequency current and a medium-frequency current are applied to the locations between the electrode sections (21) and (22) corresponding to the zygomatic muscle and between the electrode sections (21) and (23) corresponding to the masseter muscle, respectively. Patent Literature 5 disclosed a prior art in which a configuration for EMS that applies a current of a frequency of 15 Hz suitable for training muscles and a frequency of 33 Hz suitable for relaxing muscles.

In the present embodiment, a function to apply a medium-frequency current of about 1 kHz to provide muscle-relaxing treatment. A major difference in this embodiment from the prior art is that the electrode sections (21), (22), and (23) having a large area are adopted. The area of the electrode thereof is 2.5 times larger than that of Patent Literature 5, and the electrode can provide more intense sensation even at a small output level. As a result, the configuration can bring adequate beauty effect while suppressing irritation on skin.

The areas of the electrode sections (21), (22), and (23) in contact with the skin surface are about 504 square mm each, and the area of a pair of the electrodes totals about 1008 square mm. It is enlarged 2.38 times in the present invention while the area was about 423 square mm in the conventional configuration. By extending the electrode sections, sufficient area of the electrode sections is secured, and a preferable range is 300 square mm to 700 square mm per electrode section.

When subjects wear the cosmetic mask of the configuration according to the present invention, it is confirmed that the degree of facial muscle movement they felt increased by 1.3 time approximately, from 4.9 to 6.4 points when evaluated on a 7-point scale. In the present invention, the electrode parts (21), (22), and (23) are extended from the current generator (20) to ensure a sufficient contact area, thereby sufficiently improving the cosmetic effect.

Further, by extending the electrode sections (21) (22) (23) in a non-symmetrical shape, the installation orientation is restricted and mis-attachment does not occur. Conventionally, the electrodes were formed in a triangular shape to make it difficult to mistake the orientation, but the present invention makes it even more obvious and easy to use.

The first electrode section (21) and the second electrode section (22) form an angle of 130 degrees (preferable range of 120 to 140 degrees), and the first electrode section (21) and the third electrode section (23) form an angle of 160 degrees (preferable range 150 to 180 degrees). These angles constitute electrode sections that provide adequate stimulation to both the masseter and zygomatic muscles.

As described above, the present invention includes the first electrode (21) that extends toward the temporomandibular joint, and the second electrode (22) that extends toward the opening of the mouth periphery, and the third electrode (23) extending toward the mandible when the mask is worn, but it may be configured to extend in any two directions. Further, the electrode sections (21), (22), and (23) may be configured to radially extend in multiple directions.

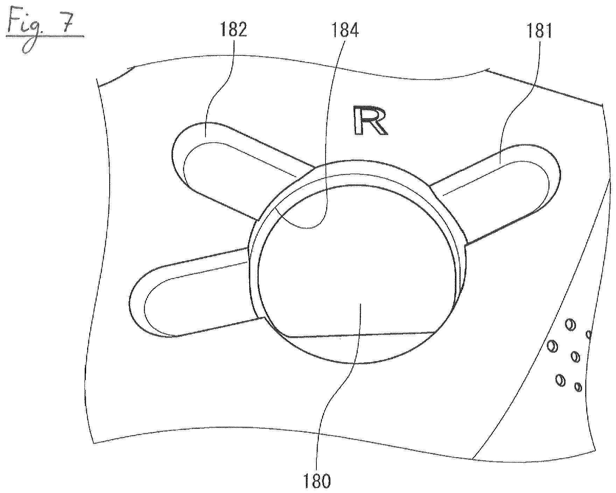

Fig. 7 shows an aspect of the stimulator fitting section (18) on the skin contact side. The stimulator fitting section (18) includes a current generator region (180) in which the current generator (20) is fitted and a part of the current generator (20) is exposed to the front surface, a first electrode region (181) in which the first electrode section (21) is fitted, a second electrode region (182) in which the second electrode section (22) is fitted, and a third electrode region (183) in which the third electrode section (23) is fitted.

The electrode regions (181), (182), and (183) do not penetrate to the front surface, and the sides of the electrode sections (21), (22), and (23) are loosely fitted to the inner periphery. On the other hand, in the current generator region (180), a groove (184) is formed over the entire periphery of the inner periphery, and the outer periphery of the current generator (20) is fixed while being fitted into the groove (184).

The stimulator (2) can be removed from the stimulator fitting section (18) to recharge or rinse. When the electrode sections (21), (22), and (23) are totally removed, the mask body (1) made of mere silicon material-makes it easy to wash and maintain. At the same time, the manufacturing cost of the mask body (1) is reduced, replacement is easy.

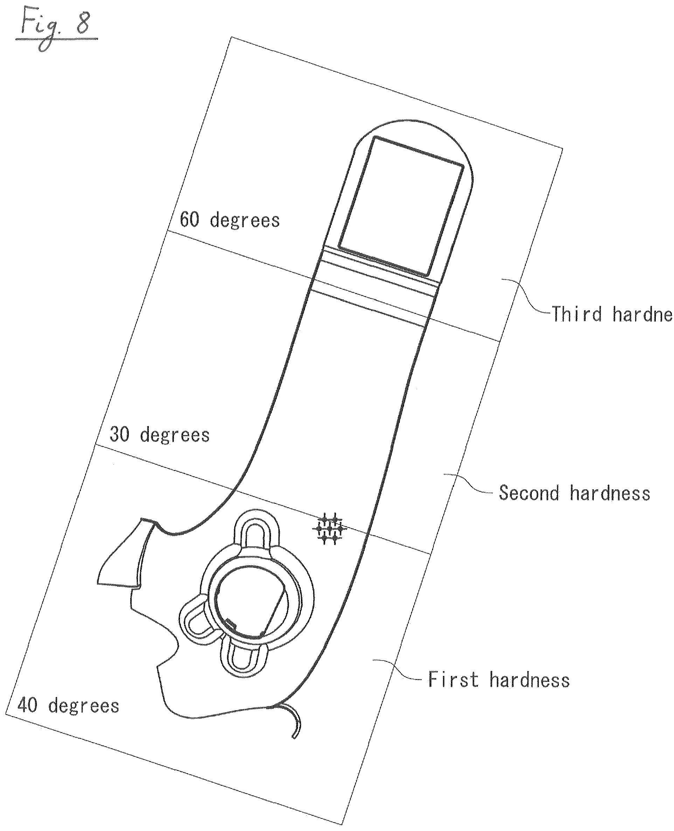

Finally, in the present invention, silicon materials having different hardness are used for each location of the mask body (1). As illustrated in Fig. 8, the location covering the lower half of the face, including the mouth periphery (10), the mandible section (11), the cheek section (12), and the nosepiece (14) have a first hardness, for example a hardness of 40 degrees to achieve both shape stability and conformity to the face.

Further, the region of the wrap-around section (13) that is stretched toward the occiput is set to a hardness lower than the first hardness, for example, 30 degrees. Being elastic, it can be wrapped around the occiput without deforming the part contacting with the face. Furthermore, the hook-and-loop fastener (130) section has a third hardness higher than the first and second hardness, e.g. 60 degrees, considering joining with the hook-and-loop fastener and making it easy to pull.

Such a distribution of hardness makes it possible to provide a cosmetic mask that is easy and comfortable to wear.

The present invention may provide the stimulator (2) as a stand-alone unit, that is equipped with two or more electrode sections radially extending from a current generator, and the current generator which comprises a control unit for controlling the current and a power supply section that supplies electricity.

The configuration of electrode sections extending radially from the current generator allows for sufficient distance between electrodes, and the area of each electrode can also be increased. Further, since the direction of radiation can be freely designed, an optimal arrangement is achieved in conform with the location of the target muscle.

When the stimulator is configured as a stand-alone unit, the electrode sections are preferably formed in a curving shape curved from the base of the current generator, so that it fits better to the skin contact surface.

### Reference Signs List

- 1: Mask body
- 10: Mouth periphery
- 11: Mandible section
- 12: Cheek section
- 13: Wrap-Around section
- 130: Hook-and-Loop fastener
- 14: Nosepiece
- 15: Upper edge
- 16: Ear section
- 17: Tab
- 18: Stimulator attachment
- 180: Current generator region
- 181, 182, 183: Electrode region
- 184: Groove
- 2: Stimulator
- 20: Current generator
- 200, 201: Adjustment button
- 202, 203: Power supply terminal
- 210: Internal electrode
- 21: Electrode section
- 22: Electrode section
- 23: Electrode section

## Claims

1. A cosmetic mask worn on the face of a user, made of a flexible elastic material, and formed in a substantial band shape to be wrapped around the occiput from a chin tip through right and left cheeks, the cosmetic mask comprising:
a mouth periphery (10) that covers at least a location corresponding to the mouth with an opening;
a mandible section (11) that covers the chin tip and at least a part of a lower surface of the mandible;
a cheek section (12) that covers a part of the cheek;
a wrap-around section (13) that is wrapped around the facial lateral sides and the occiput;
a pair of stimulators (2) that provides electric or ultrasound stimulation to a location corresponding to at least one of a zygomatic muscle and a masseter muscle of the user; and
a stimulator fitting section (18) that fits each stimulator (2) in the cheek section,
**characterized in**
**that** the stimulator (2) that provides electric stimulation includes a current generator (20) that generates an electric current and two or more electrode sections (21, 22, 23) radially extending from the current generator (20), and the current generator (20) includes a controller that controls a current and a power supply section that supplies electricity.

2. The cosmetic mask according to claim 1, wherein, in the cosmetic mask, the wrap-around section is split at the top of the occiput, and can be fastened by a hook-and-loop fastener (130).

3. The cosmetic mask according to claim 1 or 2, wherein from the width in lateral view of the face from the lower edge of the opening in the mouth periphery to the neck-side end of the mandible, the cheek and the wrap-around portion are all equal width in lateral view, respectively.

4. The cosmetic mask according to any one of claims 1 to 3, wherein a nosepiece (14) matching at least a convex shape of a nose is provided above the mouth periphery.

5. The cosmetic mask according to any one of claims 1 to 4, wherein a tab is provided at the neck-side end of the above-mentioned mandible section to pull it toward the neck when the cosmetic mask is worn.

6. The cosmetic mask according to one of claims 1 to 5, wherein the electrode sections (21, 22, 23) include
a first electrode extending in the direction of the temporomandibular joint when the cosmetic mask is worn,
a second electrode extending in the direction of the opening, and
a third electrode extending in the direction of the mandible section.

7. The cosmetic mask according to one of claims 1 to 6, wherein the electrode sections (21, 22, 23) are formed to be curved from a base from the current generator (20) toward a skin contact surface.

8. The cosmetic mask according to any one of claims 1 to 7, wherein the stimulator fitting section includes
a current generator region (180) in which the current generator (20) is fitted and at least a part of the current generator (20) is exposed to a front surface, and
an electrode region (181, 182, 183) in which corresponding one of the electrode sections (21, 22, 23) is fitted and exposed only to the skin contact surface.

9. The cosmetic mask according to any one of claims 1 to 8, wherein the cosmetic mask is integrally molded with a resin material, and hardness of the material is changed according to the location.

## Patentansprüche

1. Kosmetische Maske, die von einem Benutzer im Gesicht getragen wird, aus einem flexiblen, elastischen Material besteht und im Wesentlichen in Form eines Bandes ausgebildet ist, das von der Kinnspitze über die rechte und linke Wange um den Hinterkopf gewickelt wird, wobei die kosmetische Maske Folgendes umfasst:
eine Mundperipherie (10), die mindestens eine dem Mund entsprechende Stelle mit einer Öffnung bedeckt;
einen Unterkieferabschnitt (11), der die Kinnspitze und mindestens einen Teil einer unteren Fläche des Unterkiefers bedeckt;
einen Wangenabschnitt (12), der einen Teil der Wange bedeckt;
einen Umwickelungsabschnitt (13), der um die seitlichen Gesichtspartien und den Hinterkopf gewickelt wird;
ein Paar Stimulatoren (2), die elektrische oder Ultraschallstimulation an einer Stelle bereitstellen, die mindestens einem der folgenden entspricht: Jochbeinmuskel und Kaumuskel des Benutzers; und
einen Stimulator-Anpassungsabschnitt (18), der jeden Stimulator (2) in den Wangenabschnitt einpasst,
**dadurch gekennzeichnet,**
**dass** der Stimulator (2), der elektrische Stimulation bereitstellt, einen Stromgenerator (20), der elektrischen Strom erzeugt, und zwei oder mehr Elektrodenabschnitte (21, 22, 23) umfasst, die sich radial vom Stromgenerator (20) erstrecken, und der Stromgenerator (20) eine Steuerung umfasst, die einen Strom steuert, und einen Stromversorgungsabschnitt, der Elektrizität liefert.

2. Kosmetische Maske nach Anspruch 1, wobei in der kosmetischen Maske der Umwickelungsabschnitt am oberen Ende des Hinterkopfes geteilt ist und durch einen Klettverschluss (130) befestigt werden kann.

3. Kosmetische Maske nach Anspruch 1 oder 2, wobei in der seitlichen Ansicht des Gesichts von der Unterkante der Öffnung in der Mundperipherie bis zum halsseitigen Ende des Unterkiefers der Wangenabschnitt und der Umwicklungsabschnitt jeweils alle gleich breit sind.

4. Kosmetische Maske nach einem der Ansprüche 1 bis 3, wobei ein Nasenstück (14), das mindestens einer konvexen Form einer Nase entspricht, über der Mundperipherie vorgesehen ist.

5. Kosmetische Maske nach einem der Ansprüche 1 bis 4, wobei am halsseitigen Ende des oben erwähnten Unterkieferabschnitts eine Lasche vorgesehen ist, um sie beim Tragen der kosmetischen Maske zum Hals hin zu ziehen.

6. Kosmetische Maske nach einem der Ansprüche 1 bis 5, wobei die Elektrodenabschnitte (21, 22, 23) umfassen
eine erste Elektrode, die sich in Richtung des Kiefergelenks erstreckt, wenn die kosmetische Maske getragen wird,
eine zweite Elektrode, die sich in Richtung der Öffnung erstreckt, und
eine dritte Elektrode, die sich in Richtung des Unterkieferabschnitts erstreckt.

7. Kosmetische Maske nach einem der Ansprüche 1 bis 6, wobei die Elektrodenabschnitte (21, 22, 23) so geformt sind, dass sie von einer Basis des Stromgenerators (20) zu einer Hautkontaktfläche hin gekrümmt sind.

8. Kosmetische Maske nach einem der Ansprüche 1 bis 7, wobei der Stimulator-Anpassungsabschnitt umfasst
einen Stromgeneratorbereich (180), in dem der Stromgenerator (20) eingepasst ist und mindestens ein Teil des Stromgenerators (20) einer Vorderseite ausgesetzt ist, und
einen Elektrodenbereich (181, 182, 183), in dem ein entsprechender der Elektrodenabschnitte (21, 22, 23) eingepasst ist und nur der Hautkontaktfläche ausgesetzt ist.

9. Kosmetische Maske nach einem der Ansprüche 1 bis 8, wobei die kosmetische Maske integral mit einem Harzmaterial geformt ist und die Härte des Materials je nach Lage verändert ist.

## Revendications

1. Masque cosmétique porté sur le visage d'un utilisateur, constitué d'un matériau élastique flexible, et formé en forme de bande substantielle pour être enroulé autour de l'occiput à partir d'une pointe de menton à travers les joues droites et gauches, le masque cosmétique comprenant :
un pourtour de bouche (10) qui couvre au moins un emplacement correspondant à la bouche avec une ouverture ;
une section mandibulaire (11) qui couvre la pointe de menton et au moins une partie de la surface inférieure de la mandibule ;
une section de joue (12) qui couvre une partie de la joue ;
une section enveloppante (13) qui s'enroule autour des côtés latéraux du visage et de l'occiput ;
une paire de stimulateurs (2) qui fournit une stimulation électrique ou par ultrasons à un emplacement correspondant à au moins un des muscles zygomatiques et
un des muscles masséters de l'utilisateur ; et
une section d'adaptation de stimulateur (18) qui s'adapte à chaque stimulateur (2) dans la section de joue,
**caractérisé en ce que**
le stimulateur (2) qui fournit une stimulation électrique comprend un générateur de courant (20) qui génère un courant électrique et deux ou plus de deux sections d'électrodes (21, 22, 23) s'étendant radialement à partir du générateur de courant (20), et le générateur de courant (20) comprend un dispositif de commande qui commande un courant et une section d'alimentation qui fournit de l'électricité.

2. Masque cosmétique selon la revendication 1, dans le masque cosmétique, la section enveloppante étant divisée au sommet de l'occiput et pouvant être fixée par une fermeture auto-agrippante (130).

3. Masque cosmétique selon la revendication 1 ou 2, la largeur en vue latérale du visage, depuis le bord inférieur de l'ouverture dans le pourtour de bouche jusqu'à l'extrémité de la mandibule côté cou, la joue et la partie enveloppante étant toutes de largeur égale en vue latérale, respectivement.

4. Masque cosmétique selon l'une quelconque des revendications 1 à 3, un embout nasal (14) correspondant au moins à la forme convexe d'un nez étant prévu au-dessus du pourtour de bouche.

5. Masque cosmétique selon l'une quelconque des revendications 1 à 4, une languette étant prévue à l'extrémité côté cou de la section mandibulaire susmentionnée pour la tirer vers le cou lorsque le masque cosmétique est porté.

6. Masque cosmétique selon l'une quelconque des revendications 1 à 5, les sections d'électrodes (21, 22, 23) comprenant
une première électrode s'étendant dans la direction de l'articulation temporo-mandibulaire lorsque le masque cosmétique est porté,
une deuxième électrode s'étendant dans la direction de l'ouverture, et
une troisième électrode s'étendant dans la direction de la section mandibulaire.

7. Masque cosmétique selon l'une quelconque des revendications 1 à 6, les sections d'électrodes (21, 22, 23) étant formées pour être incurvées à partir d'une base du générateur de courant (20) vers une surface de contact avec la peau.

8. Masque cosmétique selon l'une quelconque des revendications 1 à 7, la section d'adaptation de stimulateur comprenant
une zone de générateur de courant (180) dans laquelle le générateur de courant (20) est monté et au moins une partie du générateur de courant (20) est exposée à une surface frontale, et
une zone d'électrodes (181, 182, 183) dans laquelle l'une des sections d'électrodes correspondantes (21, 22, 23) est montée et exposée uniquement à la surface de contact avec la peau.

9. Masque cosmétique selon l'une quelconque des revendications 1 à 8, le masque cosmétique étant moulé d'un seul tenant avec un matériau en résine, et la dureté du matériau étant modifiée en fonction de l'emplacement.
